(19)

**Europäisches Patentamt**
**European Patent Office**
**Office européen des brevets**

(11)  **EP 4 729 622 A1**

(12)  # EUROPEAN PATENT APPLICATION
published in accordance with Art. 153(4) EPC

(43) Date of publication:
**22.04.2026  Bulletin 2026/17**

(21) Application number: **24823488.2**

(22) Date of filing: **14.06.2024**

(51) International Patent Classification (IPC):
*C12P 1/04* (2006.01)       *A23J 3/34* (2006.01)
*A23L 5/00* (2016.01)       *A23L 33/175* (2016.01)
*C12N 9/52* (2006.01)       *C12N 9/80* (2006.01)
*C12P 13/04* (2006.01)

(52) Cooperative Patent Classification (CPC):
**A23J 3/34; A23L 5/00; A23L 33/175; C12N 9/52;**
**C12N 9/80; C12P 1/04; C12P 13/04**

(86) International application number:
**PCT/JP2024/021775**

(87) International publication number:
**WO 2024/257875 (19.12.2024 Gazette 2024/51)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **14.06.2023  JP 2023098145**

(71) Applicant: **Amano Enzyme Inc.**
**Nagoya-shi**
**Aichi 460-8630 (JP)**

(72) Inventors:
• **SAKAI, Kiyota**
**Kakamigahara-shi, Gifu 509-0109 (JP)**
• **HAYAKAWA, Mari**
**Kakamigahara-shi, Gifu 509-0109 (JP)**
• **OGAWA, Midori**
**Kakamigahara-shi, Gifu 509-0109 (JP)**
• **OKUDA, Keita**
**Kakamigahara-shi, Gifu 509-0109 (JP)**

(74) Representative: **Vossius & Partner**
**Patentanwälte Rechtsanwälte mbB**
**Siebertstraße 3**
**81675 München (DE)**

(54)  ## MODIFIER FOR PROTEIN-CONTAINING COMPOSITION

(57)    The purpose of the present invention is to provide a protein-containing composition having an improved essential amino acid release amount. By using a protein deamidase and a protease derived from the genus Chryseobacterium, it is possible to improve the essential amino acid release amount, foamability, foam stability, and/or emulsifiability of a protein-containing composition.

**EP 4 729 622 A1**

**Description**

Technical Field

The present invention relates to a processing technique for modification of a protein-containing composition.

Background Art

Protein is one of important nutrients as a component that makes a body. Efficiency of protein intake is synonymous with efficiency of digestion to amino acids. That is, efficient digestion is essential to effectively incorporate proteins into the body.

It is predicted that the world population will reach 9.7 billion in 2050, and it is feared that the future protein demand will exceed the supply (protein crisis). Under such circumstances, a movement of replacing animal proteins by plant proteins with less environmental burden is increasingly accelerated.

However, plant proteins are known to be inferior in digestibility to animal proteins (NPL 1), and a gap between food intake and nutrient absorption is also a problem.

Meanwhile, in the food industry, modification of protein materials is attracting increasing attention in terms of increasing the added value of food. Such modifications include not only improvement in digestibility for plant protein materials but also improvement in properties such as foamability, foam stability, and emulsifiability for protein materials including animal protein materials.

Citation List

Non Patent Literature

NPL 1: Journal of Japanese Society of Food and Nutrition, Vol. 20, No. 4, p. 259-266

Summary of Invention

Technical Problem

An object of the present invention is to provide a technique for producing a modified protein-containing composition. As described above, since a plant protein is inferior in digestibility to an animal protein, a first object of the present invention is to provide a technique for producing a plant protein-containing composition with an improved essential amino acid release amount. A second object of the present invention is to provide a technique for producing a protein-containing composition with improved foamability, foam stability, or emulsifiability.

Solution to Problem

The present inventors have conducted intensive studies, and as a result, have found that by performing a treatment of causing a protein deamidase and a protease derived from the genus Chryseobacterium to act on a plant protein material, an essential amino acid release amount of a plant protein-containing food and drink to be obtained is improved, and by performing a treatment of causing a protein deamidase and a protease derived from the genus Chryseobacterium to act on a protein material, the foamability, foam stability, or emulsifiability of a protein material to be obtained is improved. The present invention has been completed by conducting further studies based on these findings.

That is, the present invention provides inventions of the following aspects.

(A) A modifier for a protein-containing composition, the modifier containing a protein deamidase and a protease derived from the genus Chryseobacterium, in which the modification of the protein-containing composition is an improvement in an essential amino acid release amount of a plant protein-containing composition, foamability of the protein-containing composition, foam stability of the protein-containing composition, and/or emulsifiability of the protein-containing composition.

A use of a combination of a protein deamidase and a protease derived from the genus Chryseobacterium for modifying a protein-containing composition, in which the modification of the protein-containing composition is an improvement in an essential amino acid release amount of a plant protein-containing composition, foamability of the protein-containing composition, foam stability of the protein-containing composition, and/or emulsifiability of the protein-containing composition.

An application of a combination of a protein deamidase and a protease derived from the genus Chryseobacterium as a modifier for a protein-containing composition, in which the modification of the protein-containing composition is an improvement in an essential amino acid release amount of a plant protein-containing composition, foamability of the protein-containing composition, foam stability of the protein-containing composition, and/or emulsifiability of the protein-containing composition.

Item 1. An agent for improving an essential amino acid release amount of a plant protein-containing composition, the agent containing a protein deamidase and a protease derived from the genus Chryseobacterium.

Item 2. The agent for improving an essential amino acid release amount of a plant protein-containing composition described in item 1, in which the protein deamidase is a protein glutaminase.

Item 3. The agent for improving an essential amino acid release amount of a plant protein-containing composition described in item 1 or 2, in which the plant protein is a protein of a plant selected from the group consisting of pulses, cereals, and nuts and seeds.

Item 4. A modifier for a protein-containing composition, the modifier containing a protein deamidase and a protease derived from the genus Chryseobacterium, in which the modification is an improvement in foamability, foam stability, and/or emulsifiability.

(B) A modification method for a protein-containing composition, the modification method including a step of causing a protein deamidase and a protease derived from the genus Chryseobacterium to act on a protein-containing composition, in which the modification of the protein-containing composition is an improvement in an essential amino acid release amount of a plant protein-containing composition, foamability of the protein-containing composition, foam stability of the protein-containing composition, and/or emulsifiability of the protein-containing composition.

Item 5. A method for improving an essential amino acid release amount of a plant protein-containing composition, the method including a step of causing a protein deamidase and a protease derived from the genus Chryseobacterium to act on a plant protein-containing composition.

Item 6. A modification method for a protein-containing composition, the modification method including a step of causing a protein deamidase and a protease derived from the genus Chryseobacterium to act on a protein-containing composition, in which the modification is an improvement in foamability, foam stability, and/or emulsifiability.

(C) A method for producing a processed protein-containing composition, the method including a step of causing a protein deamidase and a protease derived from the genus Chryseobacterium to act on a protein-containing composition, in which the processed protein-containing composition is any of a processed plant protein-containing composition with an improved essential amino acid release amount and a processed protein-containing composition with improved foamability, foam stability, and/or emulsifiability.

Item 7. A method for producing a processed plant protein-containing composition with an improved essential amino acid release amount, the method including a step of causing a protein deamidase and a protease derived from the genus Chryseobacterium to act on a plant protein-containing composition.

Item 8. A method for producing a processed protein-containing composition with improved foamability, foam stability, and/or emulsifiability, the method including a step of causing a protein deamidase and a protease derived from the genus Chryseobacterium to act on a protein-containing composition.

(D) A processed protein-containing composition obtained by a method including a step of causing a protein deamidase and a protease derived from the genus Chryseobacterium to act on a protein-containing composition.

Item 9. A processed plant protein-containing composition with an improved essential amino acid release amount, the processed plant protein-containing composition being obtained by a method including a step of causing a protein deamidase and a protease derived from the genus Chryseobacterium to act on a plant protein-containing composition.

Item 10. A processed protein-containing composition with improved foamability, foam stability, and/or emulsifiability, the processed protein-containing composition being obtained by a method including a step of causing a protein deamidase and a protease derived from the genus Chryseobacterium to act on a protein-containing composition.

Advantageous Effects of Invention

According to the present invention, there is provided a technique for producing a plant protein-containing composition with an improved essential amino acid release amount or a protein-containing composition with improved foamability, foam stability, or emulsifiability.

Description of Embodiments

1. Modifier for Protein-Containing Composition

Among modifiers for a protein-containing composition of the present invention, an agent for improving an essential amino acid release amount of a plant protein-containing composition (hereinafter, also referred to as "first modifier") contains a protein deamidase and a protease derived from the genus Chryseobacterium. Among modifiers for a protein-containing composition of the present invention, a modifier used for improving the foamability, foam stability, and/or emulsifiability of a protein-containing composition (hereinafter, also referred to as "second modifier") also contains a protein deamidase and a protease derived from the genus Chryseobacterium. Hereinafter, the first modifier and the second modifier are also collectively referred to as a "modifier".

1-1. Use Application

The agent for improving an essential amino acid release amount of a plant protein-containing composition according to the first modifier of the present invention is used for an application in which the essential amino acid release amount contained in the plant protein-containing composition is improved.

Specifically, the first modifier of the present invention is used for an application in which the essential amino acid amount is more increased than the non-essential amino acid amount among free amino acids. The improvement in essential amino acid release amount can be confirmed by the fact that the ratio of the amount of the essential amino acids to the total amount of the free amino acids is increased as compared with in the case of not using a combination of a protein deamidase and a protease other than a protease derived from the genus Chryseobacterium (that is, the case of treating a plant protein-containing composition under the same conditions except that a combination of a protein deamidase and a protease other than a protease derived from the genus Chryseobacterium is not used), particularly the case of using a protein deamidase and a protease other than a protease derived from the genus Chryseobacterium (that is, the case of treating a plant protein-containing composition under the same conditions except that a protein deamidase and a protease other than a protease derived from the genus Chryseobacterium are used).

The second modifier of the present invention is used for an application in which the foamability, foam stability, and/or emulsifiability of the protein-containing composition is improved.

The improvement in foamability can be confirmed by the fact that the total volume including the foam of the protein-containing composition foamed by the homogenization treatment is increased as compared with the case of not using a combination of a protein deamidase and a protease other than a protease derived from the genus Chryseobacterium (that is, the case of treating a protein-containing composition under the same conditions except that a combination of a protein deamidase and a protease other than a protease derived from the genus Chryseobacterium is not used); the improvement in foam stability can be confirmed by the fact that the degree of temporal decrease in the total volume of the foamed protein-containing composition is suppressed as compared with the above case; and the improvement in emulsifiability can be confirmed by the fact that the turbidity of the emulsion composition prepared using the protein-containing composition and the oily base is increased as compared with the above case.

The plant protein-containing composition to which the first modifier of the present invention is applied, the protein-containing composition to which the second modifier of the present invention is applied, and specific methods of using these modifiers will be described in detail in "2. Modification Method for Protein-Containing Composition".

1-2. Protein Deamidase

The protein deamidase is an active ingredient in the modifier of the present invention together with a protease derived from the genus Chryseobacterium. That is, the protein deamidase is an active ingredient that contributes to an improvement in the essential amino acid release amount in the agent for improving an essential amino acid release amount of a plant protein-containing composition according to the first modifier of the present invention, and is an active ingredient that contributes to an improvement in the foamability, foam stability, and/or emulsifiability in the second modifier of the present invention.

The type, origin, and the like of the protein deamidase are not particularly limited as long as the protein deamidase is an enzyme that exhibits an action of decomposing an amide group-containing side chain of a protein without cleaving peptide bonds and without crosslinking the protein. Examples of the protein deamidase include a protein deamidase derived from the genus Chryseobacterium, Flavobacterium, Empedobacter, Sphingobacterium, Aureobacterium, or Myroides, which is disclosed in Japanese Patent Laid-open Publication Nos. 2000-50887 and 2001-218590, and WO 2006/075772 A. These protein deamidases may be used singly or in combination of a plurality of kinds thereof.

Examples of the protein deamidase include a protein glutaminase and a protein asparaginase, and in a broad sense, a protein arginine deiminase is also included. Among these protein deamidases, a protein glutaminase is preferable from the viewpoint of further enhancing the essential amino acid release amount, foamability, foam stability, and/or emulsifiability.

Among these protein deamidases, from the viewpoint of further enhancing the essential amino acid release amount, foamability, foam stability, and/or emulsifiability, a protein deamidase derived from the genus Chryseobacterium is more preferable, a protein glutaminase derived from the genus Chryseobacterium is further preferable, and a protein glutaminase derived from Chryseobacterium proteolyticum species is still more preferable.

The protein deamidase can be prepared from a culture broth of a microorganism from which the protein deamidase is derived. Specific examples of the preparation method include a method of recovering a protein deamidase from a culture broth or a bacterial cell of the above-mentioned microorganism. For example, in the case of using a microorganism that secretes a protein deamidase, an enzyme can be separated and/or purified after recovering bacterial cells from the culture broth in advance by filtration, a centrifugal treatment, or the like, as necessary. In the case of using a microorganism that does not secrete protein deamidase, an enzyme can be separated and/or purified after recovering bacterial cells from the culture broth in advance as necessary and then disrupting the bacterial cells by a pressurization treatment, an ultrasonic treatment, or the like to expose an enzyme. As an enzyme separation and/or purification method, a known protein separation and/or purification method can be used without particular limitation, and examples thereof include such as a centrifugal separation method, a UF concentration method, a salting-out method, and various chromatography methods using an ion-exchange resin or the like. The separated and/or purified enzyme can be powdered by a drying method such as freeze-drying or reduced-pressure drying, and can also be powdered using an appropriate excipient and/or drying aid in the drying method. The separated and/or purified enzyme can also be liquefied by adding an appropriate additive and performing filtration sterilization. As the protein deamidase, a commercially available product can also be used.

1-3. Protease Derived from Genus Chryseobacterium

The protease derived from the genus Chryseobacterium is an active ingredient in the modifier of the present invention together with the protein deamidase. That is, the protease derived from the genus Chryseobacterium is an active ingredient that contributes to an improvement in the essential amino acid release amount in the agent for improving an essential amino acid release amount of a plant protein-containing composition according to the first modifier of the present invention, and is an active ingredient that contributes to an improvement in the foamability, foam stability, and/or emulsifiability in the second modifier of the present invention.

The protease derived from the genus Chryseobacterium is not particularly limited as long as it is an enzyme derived from the genus Chryseobacterium that hydrolyzes a peptide bond of a protein.

Specific examples of the protease derived from the genus Chryseobacterium include proteases derived from Chryseobacterium nematophagum, Chryseobacterium cucumeris, Chryseobacterium lactis, Chryseobacterium rhizoplanae, Chryseobacterium joostei, Chryseobacterium shigense, Chryseobacterium proteolyticum, Chryseobacterium gleum, Chryseobacterium soli, and the like. These proteases derived from the genus Chryseobacterium may be used singly or in combination of a plurality of kinds thereof. Among these proteases of the genus Chryseobacterium, from the viewpoint of further enhancing the effect of improving the essential amino acid release amount, foamability, foam stability, and/or emulsifiability, a protease derived from Chryseobacterium proteolyticum species is preferable.

The protease derived from the genus Chryseobacterium can be prepared by a known method. As an example, the protease can be easily prepared by a method of culturing bacteria belonging to the genus Chryseobacterium and separating the protease using a known means, a method using a genetic recombination technique, or the like.

1-4. Other Components

The modifier of the present invention may be an enzyme composition containing a protein deamidase and a protease derived from the genus Chryseobacterium, and may or may not contain, as components other than the enzyme, an additive and/or a base that are/is formulary acceptable for an enzyme agent. Examples of such an additive and a base include such as an excipient, a buffer, an antioxidant, an ultraviolet inhibitor, a preservative, an antiseptic, a pH adjusting agent, a dispersant, an emulsifier, a solubilizing agent, a carrier, and a solvent (water or the like). These additives and bases may be used singly or in combination of two or more kinds thereof. The contents of these additives and bases may be appropriately set according to the type and/or preparation form and the of these components.

1-5. Aspect

The aspect of the modifier for a protein-containing composition of the present invention is not particularly limited, and examples thereof include powdered, fine-grained, granular dry preparations, and liquid preparations.

2. Modification Method for Protein-Containing Composition

As described above, the combination of the protein deamidase and the protease derived from the genus Chryseobacterium contributes to the improvement in the essential amino acid release amount of the plant protein-containing composition and also contributes to the improvement in the foamability, foam stability, and/or emulsifiability of the protein-containing composition. Therefore, the present invention also provides a method for improving an essential amino acid release amount of a plant protein-containing composition, the method including a step of causing a protein deamidase and a protease derived from the genus Chryseobacterium to act on a plant protein-containing composition (hereinafter, also referred to as "first modification method"), and a modification method for a protein-containing composition, the modification method including a step of causing a protein deamidase and a protease derived from the genus Chryseobacterium to act on a protein-containing composition, in which the modification is an improvement in foamability, foam stability, and/or emulsifiability (hereinafter, also referred to as "second modification method"). Hereinafter, the first modification method and the second modification method are also collectively referred to as "modification method".

In the method for improving an essential amino acid release amount of a plant protein-containing composition according to the first modification method of the present invention, the "improvement in essential amino acid release amount", the "protein deamidase", and the "protease derived from the genus Chryseobacterium" are as described in detail in the above "1. Modifier for Protein-Containing Composition". In the second modification method of the present invention, the "improvement in foamability, foam stability, and/or emulsifiability", the "protein deamidase", and the "protease derived from the genus Chryseobacterium" are also as described in detail in the above "1. Modifier for Protein-Containing Composition".

2-1. Step of Causing Protein deamidase and Protease Derived from Genus

Chryseobacterium to Act on Protein-Containing Composition

In the step of causing a protein deamidase and a protease derived from the genus Chryseobacterium to act on a plant protein-containing composition in the first modification method, a plant protein mixture containing a plant protein-containing composition, a protein deamidase, and a protease derived from the genus Chryseobacterium is appropriately prepared, and a treatment for allowing enzymatic reaction to proceed is performed. In the step of causing a protein deamidase and a protease derived from the genus Chryseobacterium to act on a protein-containing composition in the second modification method, a protein mixture containing a protein-containing composition, a protein deamidase, and a protease derived from the genus Chryseobacterium is appropriately prepared, and a treatment for allowing enzymatic reaction to proceed is performed. In the second modification method, the protein includes a plant protein and an animal protein.

2-1-1. Plant Protein-Containing Composition

The plant protein-containing composition is an enzyme treatment target in the first modification method, and is an application target of the first modifier. The plant protein-containing composition is an example of an enzyme treatment target in the second modification method, and is an example of an application target of the second modifier. The plant protein-containing composition is not particularly limited as long as it contains a plant protein and is in a form that can be ingested by a living body.

Typically, the plant protein-containing composition used in the present invention contains a plant protein and water, and is typically a composition for food and drink. There is no particular restriction on the aspect of the plant protein-containing composition, and examples thereof include an aspect having fluidity, such as a liquid, a slurry, or a paste, (hereinafter, these plant protein-containing compositions of the aspect having fluidity are also collectively referred to as "liquid and the like"), and a solid. In the first modification method, the aspect of the plant protein-containing composition may be an aspect having fluidity or a solid form. In the second modification method, the aspect of the plant protein-containing composition is an aspect having fluidity.

Specific examples of the plant protein-containing composition include, as one prepared from an untextured protein material, (i) a liquid and the like obtained by dispersing, in water, dry powder of a plant protein material (specifically, at least one of a plant organ of a plant from which the plant protein is derived, and a material obtained by, for example, removing at least a part of components other than the protein from the plant organ to increase the content of the protein is exemplified; the same applies hereinafter); (ii) a liquid and the like obtained by crushing and dispersing a plant protein material in water, and removing an insoluble matter derived from a skin or the like of a plant material by any means such as centrifugation, filtration, filtration bag, or sieve, as necessary; (iii) a liquid and the like obtained by, for example, removing components other than the plant protein from the liquid and the like of the above (i) or (ii) to increase the content of the protein; (iv) a liquid obtained by mixing dry powder prepared from the liquid and the like of any one of the above (i) to (iii) with water; and preferably, in the case of using cereals as a plant protein material, (v) a liquid and the like obtained by further treating the liquid of any one of the above (i) to (iv) with amylase, and include, as one prepared from a textured protein material, (vi) a textured plant protein material swollen with water.

Note that, the textured plant protein material used in preparation of the specific example of the above (vi) is generally a food product material known as an alternative meat (pseudo meat). Typical examples of the textured plant protein material include a material in which a raw material mixture containing a plant protein and water is extruded with an extruder or the like and dried or frozen to be textured like meat. Note that, in the present invention, the "meat" imitated by the textured plant protein material means a muscle of an animal that is edible, and when described as "meat", the "meat" is used in the sense of including not only muscles of mammals and birds but also fish and shellfish.

Preferred examples of the liquid plant protein-containing composition include a so-called plant alternative milk (also referred to as plant milk).

Note that, in the following description, the "content of the plant protein material" in the plant protein-containing composition refers to, in the case of a plant protein-containing composition prepared using an untextured plant protein material, a proportion occupied by the dry weight of the component constituting the above-described plant organ contained in the plant protein-containing composition, and refers to, in the case of a plant protein-containing composition prepared using a textured plant protein material, a proportion occupied by the dry weight of the textured plant protein material. For example, when the plant protein-containing composition is a liquid and the like composed only of a component derived from a plant organ and water as in the specific examples of (i) to (v) described above, the "content of the plant protein material" refers to a proportion occupied by the dry weight of the liquid and the like. When the plant protein-containing composition is a liquid and the like containing the specific examples of (i) to (v) described above and an additive component, the "content of the plant protein material" refers to a proportion occupied by the dry weight of a portion obtained by removing the additive component from the liquid and the like. When the plant protein-containing composition is a swollen product composed only of a textured plant protein material and water as in the specific example of (vi) described above, the "content of the plant protein material" refers to a proportion occupied by the dry weight of the composition. When the plant protein-containing composition is a swollen product containing the specific example of (vi) described above and an additive component, the "content of the plant protein material" refers to a proportion occupied by the dry weight of a portion obtained by removing the additive component from the swollen product.

The content of the plant protein contained in the plant protein material is not particularly limited, and is, for example, 5 wt% or more, 10 wt% or more, 13 wt% or more, 15 wt% or more, 20 wt% or more, 25 wt% or more, or 30 wt% or more. From the viewpoint of further enhancing the effect of improving the essential amino acid release amount, the content is preferably 35 wt% or more, more preferably 40 wt% or more, and further preferably 45 wt% or more. The upper limit of the content range is not particularly limited, and is, for example, 90 wt% or less, preferably 85 wt% or less or 80 wt% or less, more preferably 70 wt% or less, further preferably 60 wt% or less, and still more preferably 55 wt% or less. The specific range of the content of the plant protein contained in the plant protein material is, for example, 5 to 90 wt% and preferably 10 to 85 wt%; when the plant protein is derived from pulses, the content is, for example, 5 to 90 wt%, preferably 30 to 85 wt%, and more preferably 40 to 85 wt%, 45 to 85 wt%, or 50 to 80 wt%; and when the plant protein is derived from cereals, the content is, for example, 5 to 90 wt% and preferably 10 to 85 wt%, 10 to 60 wt%, 10 to 40 wt%, 10 to 20 wt%, or 13 to 20 wt%.

The content of the plant protein material in the plant protein-containing composition is also not particularly limited, and is, for example, 0.05 to 80 wt%, and in the case of preparing the plant protein-containing composition from an untextured protein material, the content is, for example, 0.05 to 80 wt%, 0.1 to 60 wt%, or 1 to 40 wt%, preferably 2 to 30 wt% or 3 to 20 wt%, and more preferably 5 to 15 wt% or 8 to 12 wt%.

2-1-1-1. Plant protein

The plant protein is not particularly limited as long as its origin is a plant, and examples thereof include natural proteins contained in pulses such as soybean, pea, lentil bean, chickpea, black bean, fava bean, mung bean, lupine bean, and kidney bean; cereals such as wheat, barley, oat, sorghum, rice, rye, buckwheat, Japanese barnyard millet, foxtail millet, teff, quinoa, corn, and potato; nuts and seeds such as almond, coconut, peanut, cashew nut, hazelnut, pecan nut, macadamia nut, pistachio, walnut, brazil nut, pili nut, chestnut, sesame, pine nut, hemp seed (industrial hemp), chia seed, chia, amaranthus, canary seed, and linseed; and algae. Since the plant protein in the present invention may be any protein derived from a plant, the plant protein may be, in addition to the above natural protein, a chemically partially decomposed protein of the above-described natural protein by an acid, an alkali, or the like, an enzymatically partially decomposed protein by a protease or the like, or a chemically modified protein by various reagents, and may be a protein obtained by artificial peptide synthesis.

In the present invention, one kind of the plant protein may be used alone, or two or more kinds thereof may be used in combination. In the first modification method, from the viewpoint of further enhancing the effect of improving the essential amino acid release amount, proteins of pulses or cereals are preferable, proteins of soybean, pea, lentil bean, chickpea, black bean, fava bean, mung bean, lupine bean, kidney bean, and oat are more preferable, and proteins of soybean and pea are further preferable. In the second modification method, from the viewpoint of further enhancing the emulsifiability, proteins of cereals are preferable, and oat protein is more preferable.

The content of the plant protein in the plant protein-containing composition is not particularly limited, and is, for example, 0.01 to 50 wt%. In the first modification method, the content of the plant protein in the plant protein-containing composition is, for example, 0.01 to 50 wt%, and in the case of preparing the plant protein-containing composition from an untextured protein material, the content is, for example, 0.01 to 50 wt%, 0.1 to 40 wt%, or 0.5 to 30 wt%, preferably 1 to 25 wt% or 2 to 20 wt%, and more preferably 2.5 to 15 wt% or 3 to 10 wt%. In the second modification method, the content of the plant protein in the plant protein-containing composition is, for example, 0.01 to 50 wt%, preferably 0.1 to 40 wt%, more preferably 0.5 to 30 wt%, and further preferably 1 to 25 wt%, 1 to 20 wt%, 1 to 15 wt%, 1 to 10 wt% or 1 to 5 wt%.

## 2-1-1-2. Other Components

The plant protein-containing composition may or may not contain any components as other components in addition to the plant protein. Examples of the other components include components contained in a plant from which the plant protein is derived (carbohydrates, lipids, and the like), and additive components (other food materials, food additives, and the like). Examples of the food additives include a thickener, a binder, a seasoning, a pH adjusting agent, a buffer, a coloring agent, a flavor, and the like.

## 2-1-2. Animal Protein-Containing Composition

The animal protein-containing composition is an example of an enzyme treatment target in the second modification method, and is an example of an application target of the second modifier. The animal protein-containing composition is not particularly limited as long as it is in a form that can be ingested by a living body.

Typically, the animal protein-containing composition used in the present invention contains an animal protein, and has an aspect having fluidity such as a liquid, a slurry, or a paste. Specific examples of the animal protein-containing composition include an animal milk, a liquid obtained by dispersing a dry animal milk in water, and a liquid and the like obtained by dispersing a protein roughly purified or purified from an animal milk in water. Note that, in the following description, the "content of the animal protein material" in the animal protein-containing composition refers to the proportion of the dry weight of the animal protein-containing composition (excluding the weight of the additive component).

The content of the animal protein material in the animal protein-containing composition is not particularly limited, and is, for example, 0.05 to 40 wt%, preferably 0.1 to 30 wt% or 1 to 10 wt%, and preferably 2 to 7 wt%.

## 2-1-2-1. Animal Protein

The animal protein is not particularly limited as long as its origin is an animal, but milk protein is preferably, whey and casein are more preferable, and whey is further preferable.

The content of the animal protein in the animal protein-containing composition is not particularly limited, and is, for example, 0.03 to 30 wt%, preferably 0.08 to 20 wt% or 0.8 to 8 wt%, and preferably 1 to 6 wt%.

## 2-1-2-2. Other Components

The animal protein-containing composition may or may not contain any components as other components in addition to the animal protein. Examples of the other components include components contained in a material of origin of the animal protein (carbohydrates, lipids, and the like), and additive components (other food materials, food additives, and the like). Examples of the food additives include a thickener, a binder, a seasoning, a pH adjusting agent, a buffer, a coloring agent, a flavor, and the like.

## 2-1-3. Used amount of protein deamidase

The used amount of the protein deamidase is not particularly limited, but in the first modification method, the used amount per 1 g of the plant protein material contained in the plant protein-containing composition is, for example, 0.01 U or more or 0.1 U or more, and from the viewpoint of further enhancing the effect of improving the essential amino acid release amount, the used amount is preferably 1 U or more or 5 U or more, more preferably 7.5 U or more, further preferably 10 U or more, and still more preferably 13 U or more. The upper limit of the used amount range of the protein deamidase per 1 g of the plant protein material is not particularly limited, and is, for example, 400 U or less, 300 U or less, 200 U or less, 150 U or less, 100 U or less, or 75 U or less, preferably 60 U or less or 50 U or less, more preferably 40 U or less or 30 U or less, and further preferably 25 U or less, 20 U or less, or 17 U or less.

In the second modification method, as for the used amount of the protein deamidase, the used amount per 1 g of the plant protein material contained in the protein-containing composition is, for example, 0.01 U or more or 0.1 U or more, and from the viewpoint of further enhancing the effect of improving the foamability, foam stability, and/or emulsifiability, the used amount is preferably 1 U or more. The upper limit of the used amount range of the protein deamidase per 1 g of the plant protein material is not particularly limited, and is, for example, 100 U or less, 50 U or less, or 20 U or less, preferably 10 U or less, more preferably 5 U or less, and further preferably 3 U or less.

In the second modification method, as for the used amount of the protein deamidase, the used amount per 1 g of the animal protein material contained in the protein-containing composition is, for example, 0.1 U or more or 1 U or more, and from the viewpoint of further enhancing the effect of improving the foamability, foam stability, and/or emulsifiability, the used amount is preferably 3 U or more, more preferably 5 U or more, and further preferably 7 U or more or 8 U or more. The upper limit of the used amount range of the protein deamidase per 1 g of the animal protein material is not particularly limited, and is, for example, 400 U or less, 200 U or less, 100 U or less, 50 U or less, or 30 U or less, preferably 20 U or less, more preferably 15 U or less, and further preferably 12 U or less.

In the first modification method, the used amount of the protein deamidase per 1 g of the plant protein is, for example, 0.01 U or more or 0.1 U or more, and from the viewpoint of further enhancing the effect of improving the essential amino acid release amount, the used amount is preferably 1 U or more or 5 U or more, more preferably 7.5 U or more or 10 U or more, further preferably 13 U or more or 16 U or more, and still more preferably 18 U or more, 20 U or more, or 22 U or more. The upper limit of the used amount range of the protein deamidase per 1 g of the plant protein is not particularly limited, and is, for example, 500 U or less, 400 U or less, 300 U or less, 200 U or less, 150 U or less, 100 U or less, or 80 U or less, preferably 70 U or less or 60 U or less, more preferably 50 U or less or 45 U or less, and further preferably 40 U or less, 35 U or less, or 32 U or less. In the first modification method, the specific range of the used amount of the protein deamidase per 1 g of the plant protein is, for example, 0.01 to 500 U, 0.01 to 400 U, or 0.1 to 300 U, preferably 1 to 200 U, 5 to 150 U, or 5 to 100 U, more preferably 7.5 to 80 U or 10 to 70 U, further preferably 13 to 60, 16 to 50 U, 18 to 45 U, or 20 to 45 U, and still more preferably 22 to 40 U, 22 to 35 U, or 22 to 32 U.

In the second modification method, as for the used amount of the protein deamidase, the used amount per 1 g of the plant protein is, for example, 0.01 U or more or 0.1 U or more, and from the viewpoint of further enhancing the effect of improving the foamability, foam stability, and/or emulsifiability, the used amount is preferably 1 U or more or 3 U or more, more preferably 5 U or more or 7 U or more, and further preferably 9 U or more. The upper limit of the used amount range of the protein deamidase per 1 g of the plant protein is not particularly limited, and is, for example, 100 U or less, 50 U or less, or 20 U or less, preferably 15 U or less. In the second modification method, the specific range of the used amount of the protein deamidase per 1 g of the plant protein is, for example, 0.01 to 100 U, 0.1 to 50 U, 1 to 20 U, or 3 to 20 U, more preferably 5 to 15 U or 7 to 15 U, and further preferably 9 to 15 U.

In the second modification method, as for the used amount of the protein deamidase, the used amount per 1 g of the animal protein is, for example, 0.01 U or more or 0.1 U or more, and from the viewpoint of further enhancing the effect of improving the foamability, foam stability, and/or emulsifiability, the used amount is preferably 1 U or more or 3 U or more, more preferably 5 U or more or 7 U or more, and further preferably 9 U or more. The upper limit of the used amount range of the protein deamidase per 1 g of the animal protein is not particularly limited, and is, for example, 100 U or less, 50 U or less, or 20 U or less, preferably 15 U or less. In the second modification method, the specific range of the used amount of the protein deamidase per 1 g of the animal protein is, for example, 0.01 to 100 U, 0.1 to 50 U, 1 to 20 U, or 3 to 20 U, more preferably 5 to 15 U or 7 to 15 U, and further preferably 9 to 15 U.

Note that, for the activity of the protein deamidase, benzyloxycarbonyl-L-glutaminylglycine (Z-Gln-Gly) is used as a substrate, and the amount of enzyme that liberates 1 $\mu$mol of ammonia per minute is defined as 1 unit (1 U).

2-1-4. Used Amount of Protease Derived from Genus Chryseobacterium

The used amount of the protease derived from the genus Chryseobacterium is not particularly limited, but in the first modification method, the used amount per 1 g of the plant protein material contained in the plant protein-containing composition is, for example, 0.05 U or more, and from the viewpoint of further enhancing the effect of improving the essential amino acid release amount, the used amount is preferably 0.1 U or more or 1 U or more, more preferably 5 U or more or 7.5 U or more, further preferably 10 U or more or 12 U or more, and still more preferably 15 U or more or 18 U or more. The upper limit of the used amount range of the protease derived from the genus Chryseobacterium per 1 g of the plant protein material is not particularly limited, and is, for example, 400 U or less, 300 U or less, 200 U or less, 150 U or less, 100 U or less, or 90 U or less, preferably 80 U or less, 70 U or less, or 60 U or less, more preferably 50 U or less or 40 U or less, and further preferably 30 U or less, 25 U or less, or 22 U or less.

In the second modification method, as for the used amount of the protease derived from the genus Chryseobacterium, the used amount per 1 g of the plant protein material contained in the protein-containing composition is, for example, 0.1 U or more or 1 U or more, and from the viewpoint of further enhancing the effect of improving the foamability, foam stability, and/or emulsifiability, the used amount is preferably 2 U or more or 2.5 U or more, more preferably 5 U or more, 5.5 U or more, 6 U or more, 7 U or more, or 8 U or more, and further preferably 10 U or more or 12 U or more. The upper limit of the used amount range of the protease derived from the genus Chryseobacterium per 1 g of the plant protein material is not particularly limited, and is, for example, 400 U or less, 200 U or less, 100 U or less, 50 U or less, 30 U or less, or 20 U or less.

In the second modification method, as for the used amount of the protease derived from the genus Chryseobacterium, the used amount per 1 g of the animal protein material contained in the protein-containing composition is, for example, 0.005 U or more, and from the viewpoint of further enhancing the effect of improving the foamability, foam stability, and/or emulsifiability, the used amount is preferably 0.05 U or more, 0.5 U or more, or 1 U or more, more preferably 5 U or more or 10 U or more, further preferably 15 U or more or 20 U or more, still more preferably 35 U or more, 40 U or more, or 50 U or more, and even further preferably 70 U or more, 80 U or more, or 85 U or more. The upper limit of the used amount range of the protease derived from the genus Chryseobacterium per 1 g of the animal protein material is not particularly limited, and is, for example, 400 U or less, 200 U or less, or 100 U or less.

In the first modification method, the used amount of the protease derived from the genus Chryseobacterium per 1 g of the plant protein is, for example, 0.05 U or more, and from the viewpoint of further enhancing the effect of improving the essential amino acid release amount, the used amount is preferably 0.1 U or more or 1 U or more, more preferably 5 U or more or 7.5 U or more, further preferably 10 U or more, 12.5 U or more, 15 U or more, or 20 U or more, and still more preferably 22 U or more or 24 U or more. The upper limit of the used amount range of the protease derived from the genus Chryseobacterium per 1 g of the plant protein is not particularly limited, and is, for example, 1000 U or less, 500 U or less, 400 U or less, 300 U or less, 200 U or less, 150 U or less, 100 U or less, or 90 U or less, preferably 80 U or less, 70 U or less, or 60 U or less, more preferably 55 U or less or 50 U or less, and further preferably 45 U or less or 40 U or less. In the first modification method, the specific range of the used amount of the protease derived from the genus Chryseobacterium per 1 g of the plant protein is, for example, 0.05 to 1000 U, 0.05 to 500 U, or 0.05 to 400 U, preferably 0.1 to 300 U, 0.1 to 200 U, or 1 to 150 U, more preferably 5 to 100 U or 7.5 to 90 U, further preferably 10 to 80 U, 12.5 to 70 U, 15 to 60 U, or 20 to 55 U, and still more preferably 22 to 50 U, 22 to 45 U, or 24 to 40 U.

In the second modification method, as for the used amount of the protease derived from the genus Chryseobacterium, the used amount per 1 g of the plant protein contained in the protein-containing composition is, for example, 1 U or more or 10 U or more, and from the viewpoint of further enhancing the effect of improving the foamability, foam stability, and/or emulsifiability, the used amount is preferably 15 U or more or 20 U or more, more preferably 40 U or more, 50 U or more, 60 U or more, or 70 U or more, and further preferably 80 U or more, 90 U or more, or 100 U or more. The upper limit of the used amount range of the protease derived from the genus Chryseobacterium per 1 g of the plant protein is not particularly limited, and is, for example, 400 U or less, 300 U or less, 200 U or less, or 150 U or less. In the second modification method, the specific range of the used amount of the protease derived from the genus Chryseobacterium per 1 g of the plant protein is, for example, 1 to 400 U or 10 to 400 U, preferably 15 to 400 U or 20 to 400 U, more preferably 40 to 400 U, 50 to 400 U, 60 to 400 U, or 70 to 400 U, and further preferably 80 to 400 U, 90 to 400 U, 90 to 300 U, 90 to 200 U, or 90 to 150 U.

In the second modification method, as for the used amount of the protease derived from the genus Chryseobacterium, the used amount per 1 g of the animal protein contained in the protein-containing composition is, for example, 0.1 U or more or 1 U or more, from the viewpoint of further enhancing the effect of improving the foamability, foam stability, and/or emulsifiability, the used amount is preferably 7 U or more, 10 U or more, or 13 U or more, and more preferably 17 U or more or 20 U or more, from the viewpoint of further enhancing the effect of improving the foamability and/or emulsifiability, the used amount is further preferably 30 U or more, 40 U or more or 50 U or more, and from the viewpoint of further enhancing the effect of improving the emulsifiability, the used amount is still more preferably 60 U or more, 80 U or more, 90 U or more, or 100 U or more. The upper limit of the used amount range of the protease derived from the genus Chryseobacterium per 1 g of the animal protein is not particularly limited, and is, for example, 400 U or less, 300 U or less, 200 U or less, or 150 U or less, and from the viewpoint of improving the foam stability, the upper limit is preferably 100 U or less, 60 U or less, or 50 U or less, and further preferably 30 U or less. In the second modification method, the specific range of the used amount of the protease derived from the genus Chryseobacterium per 1 g of the animal protein is, for example, 0.1 to 400 U or 1 to 400 U, and preferably 7 to 400 U, 10 to 400 U, 10 to 200 U, 10 to 150 U, 13 to 100 U, 17 to 60 U, 20 to 50 U, 20 to 30 U, 13 to 400 U, 17 to 400 U, 20 to 400 U, 30 to 400 U, 40 to 400 U, 50 to 400 U, 60 to 400 U, 80 to 400 U, 90 to 400 U, 90 to 300 U, 90 to 200 U, or 90 to 150 U.

The used ratio of the protein deamidase and the protease derived from the genus Chryseobacterium is determined based on the above used amount for each enzyme, but from the viewpoint of further enhancing the effect of improving the essential amino acid release amount, the following used ratio is preferable.

In the first modification method, the used amount of the protease derived from the genus Chryseobacterium per 1 U of the protein deamidase is preferably 0.005 U or more, more preferably 0.05 U or more or 0.1 U or more, further preferably 0.25 U or more, 0.5 U or more, or 0.7 U or more, and still more preferably 0.8 U or more, 1 U or more, 1.1 U or more, or 1.2 U or more. The upper limit of the used amount range of the protease derived from the genus Chryseobacterium per 1 U of the protein deamidase is, for example, 20 U or less, and from the viewpoint of further enhancing the effect of improving the essential amino acid release amount, the upper limit is preferably 15 U or less or 10 U or less, more preferably 8 U or less or 6 U or less, further preferably 5 U or less or 4 U or less, and still more preferably 3 U or less, 2 U or less, or 1.5 U or less. In the first modification method, the specific range of the used amount of the protease derived from the genus Chryseobacterium per 1 U of the protein deamidase is preferably 0.005 to 20 U, more preferably 0.05 to 15 U or 0.1 to 10 U, further preferably 0.25 to 8 U, 0.5 to 6 U, or 0.7 to 5 U, and still more preferably 0.8 to 4 U, 1 to 3 U, 1.1 to 2 U, or 1.2 to 1.5 U.

In the second modification method, the used amount of the protease derived from the genus Chryseobacterium per 1 U of the protein deamidase is preferably 0.005 U or more or 0.01 U or more, from the viewpoint of further enhancing the effect of improving the foamability, foam stability, and/or emulsifiability, the used amount is more preferably 0.05 U or more, 0.1 U or more, or 0.3 U or more, further preferably 0.8 U or more, 1 U or more, or 1.3 U or more, and still more preferably 1.8 U or more or 2 U or more, from the viewpoint of further enhancing the effect of improving the foamability and/or emulsifiability, the used amount is even further preferably 2.5 U or more, 4 U or more, or 5 U or more, and from the viewpoint of further enhancing the effect of improving the emulsifiability, the used amount is particularly preferably 6 U or more, 8 U or more, or 10 U or more. The upper limit of the used amount range of the protease derived from the genus Chryseobacterium per 1 U of the protein deamidase is, for example, 50 U or less, 30 U or less, 20 U or less, or 15 U or less, and from the viewpoint of further enhancing the foam stability, the upper limit is preferably 10 U or less, more preferably 6 U or less or 5 U or less, and further preferably 3 U or less. In the second modification method, the specific range of the used amount of the protease derived from the genus Chryseobacterium per 1 U of the protein deamidase is preferably 0.005 to 50 U or 0.01 to 50 U, and more preferably 0.05 to 50 U, 0.1 to 50 U, 0.3 to 50 U, 0.8 to 50 U, 1 to 50 U, 1.3 to 50 U, 1.3 to 30 U, 1.3 to 20 U, 1.3 to 15 U, 1.3 to 10 U, 1.3 to 7 U, 1.3 to 5 U, 1.3 to 3 U, 1.8 to 50 U, 2 to 50 U, 2.5 to 50 U, 4 to 50 U, 5 to 50 U, 6 to 50 U, 8 to 50 U, 8 to 50 U, 8 to 30 U, 8 to 20 U, 8 to 15 U, or 8 to 10 U.

The protease activity is measured using casein as a substrate by the Folin method. That is, the protease activity is determined in such a manner that an enzymatic reaction is performed using casein as a substrate at a pH set according to the optimum pH of a protease to be measured by a conventional method, and an amount of an enzyme which causes an increase in colored materials by Folin's reagent corresponding to 1 $\mu$g of tyrosine per minute is defined as 1 unit (1 U).

2-1-5. Reaction Operation and Treatment Conditions

The method for preparing the plant protein mixture in the first modification method and the protein mixture in the second modification method is not particularly limited. The treatment conditions (such as temperature, time, and pH) are not particularly limited as long as the effects of the present invention can be obtained. The order of the actions of the protein deamidase and the protease derived from the genus Chryseobacterium is not particularly limited, and the enzymes may be sequentially made to act in any order, or both the enzymes may be simultaneously made to act, but preferably, both the enzymes can be simultaneously made to act.

The treatment temperature of the plant protein mixture in the first modification method and the protein mixture in the second modification method is, for example, 4 to 80°C and preferably 8 to 70°C. The lower limit of the temperature range may be 15°C, 30°C, or 45°C, and the upper limit of the temperature range may be 65°C, 60°C, or 55°C. The treatment time in the first modification method is not particularly limited, and is, for example, 0.1 to 72 hours and preferably 12 to 36 hours. The treatment time in the second modification method is also not particularly limited, and is, for example, 0.1 to 12 hours, preferably 0.5 to 6 hours, and more preferably 1 to 3 hours. The treatment pH (at 25°C) of the plant protein mixture is, for example, 2 to 9, preferably 3 to 8, more preferably 5 to 7.6, further preferably 6 to 7.4, and still more preferably 6.5 to 7.2.

These treatment conditions are appropriately selected according to the optimum temperature and optimum pH of an enzyme to be used, and/or the degree of the required effect (improvement in essential amino acid release amount, foamability, foam stability, and/or emulsifiability) of the present invention. Note that the optimum treatment conditions may be determined through a preliminary experiment.

2-2. Other Steps

The modification method of the present invention may or may not include other steps which are other than the step of causing a protein deamidase and a protease derived from the genus Chryseobacterium to act. Examples of the other steps include a step of preparing a plant protein-containing composition used in the first modification method or a protein-containing composition used in the second modification method (a plant protein-containing composition or an animal protein-containing composition), a step of inactivating an enzyme, a cooling step, a filtration step, and the like. These other steps may be performed singly or in combination of two or more steps.

The step of preparing a plant or animal protein-containing composition can be performed by any method for preparing a plant or animal protein-containing composition.

For example, in the case of using a plant alternative milk (plant milk) as the plant protein-containing composition, the plant protein-containing composition can be prepared by any method for preparing a plant alternative milk according to the type of a plant from which the plant protein is derived. In the step of preparing a plant or animal protein-containing composition, blending of an optional food additive such as a seasoning, a pH adjusting agent, a buffer, a coloring agent, or a flavor, and/or an optional treatment or the like (for example, fermentation treatment such as lactic acid fermentation) other than the treatment with a protein deamidase and a protease derived from the genus Chryseobacterium may or may not be further performed.

3. Method for Producing Processed Protein-Containing Composition

The present invention also provides a method for producing a processed plant protein-containing composition with an improved essential amino acid release amount, the production method including a step of causing a protein deamidase and a protease derived from the genus Chryseobacterium to act on a plant protein-containing composition (hereinafter, also referred to as "first production method"), and a method for producing a processed protein-containing composition with improved foamability, foam stability, and/or emulsifiability, the production method including a step of causing a protein deamidase and a protease derived from the genus Chryseobacterium to act on a protein-containing composition (hereinafter, also referred to as "second production method") Hereinafter, the first production method and the second production method are also collectively referred to as "production method".

In the first production method of the present invention, the "improvement in essential amino acid release amount", "protein deamidase", and "protease derived from the genus Chryseobacterium" are as described in detail in the above "1. Modifier for Protein-Containing Composition", and the "step of causing a protein deamidase and a protease derived from the genus Chryseobacterium to act on a plant protein-containing composition" is as described in detail in the above "2. Modification Method for Protein-Containing Composition". In the second production method of the present invention, the "improvement in foamability", "improvement in foam stability", "improvement in emulsifiability", "protein deamidase", and "protease derived from the genus Chryseobacterium" are as described in detail in the above "1. Modifier for Protein-Containing Composition", and the "step of causing a protein deamidase and a protease derived from the genus Chryseobacterium to act on a protein-containing composition" is as described in detail in the above "2. Modification Method for Protein-Containing Composition".

The production method of the present invention may or may not include other steps which are other than the step of causing a protein deamidase and a protease derived from the genus Chryseobacterium to act. In the "other steps", in addition to those described in detail in the above "2. Modification Method for Protein-Containing Composition", an optional preparing step for obtaining a final food and drink product form of a processed plant protein-containing composition to be produced can be performed.

As an example of the preparing step, for example, in the case of using a textured form as the plant protein-containing composition in the first production method, the composition after being treated with a protein deamidase and a protease derived from the genus Chryseobacterium is molded into a shape suitable for a desired form, and heat-cooked as necessary, whereby a processed plant protein-containing composition (food product) can be obtained. The method for heat-cooking can be appropriately determined by those skilled in the art according to the type of a food product. Specific examples of the heat-cooking method include boiling, dry-heat cooking (roasting, toasting, baking, grilling, or broiling), steaming, frying, and the like. These heat-cooking methods may be used singly or in combination of a plurality of kinds thereof.

4. Processed Protein-Containing Composition

The present invention also provides a processed plant protein-containing composition (hereinafter, referred to as "first processed protein-containing composition") with an improved essential amino acid release amount obtained by a method including a step of causing a protein deamidase and a protease derived from the genus Chryseobacterium to act on a plant protein-containing composition, and a processed protein-containing composition (hereinafter, referred to as "second processed protein-containing composition") with improved foamability, foam stability, and/or emulsifiability obtained by a method including a step of causing a protein deamidase and a protease derived from the genus Chryseobacterium to act on a protein-containing composition. Hereinafter, the first processed protein-containing composition and the second processed protein-containing composition are also collectively referred to as "processed protein-containing composition".

The first processed protein-containing composition of the present invention is a composition processed to improve an essential amino acid release amount by a protein deamidase and a protease derived from the genus Chryseobacterium. The second processed protein-containing composition of the present invention is a composition processed to improve foamability, foam stability, and/or emulsifiability by a protein deamidase and a protease derived from the genus Chryseobacterium.

In the first processed protein-containing composition of the present invention, the "improvement in essential amino acid release amount", "protein deamidase", and "protease derived from the genus Chryseobacterium" are as described in detail in the above "1. Modifier for Protein-Containing Composition", and the "method including a step of causing a protein deamidase and a protease derived from the genus Chryseobacterium to act on a plant protein-containing composition" is as described in detail in the above "2. Modification Method for Protein-Containing Composition" and "3. Method for Producing Processed Protein-Containing Composition". In the second processed protein-containing composition of the present invention, the "improvement in foamability", "improvement in foam stability", "improvement in emulsifiability", "protein deamidase", and "protease derived from the genus Chryseobacterium" are as described in detail in the above "1. Modifier for Protein-Containing Composition", and the "step of causing a protein deamidase and a protease derived from the genus Chryseobacterium to act on a protein-containing composition" is as described in detail in the above "2. Modification Method for Protein-Containing Composition" and "3. Method for Producing Processed Protein-Containing Composition".

The specific form of the processed protein-containing composition of the present invention can be selected from any food or drink product forms.

In the case of using a textured form as the plant protein-containing composition that is a material for the first processed protein-containing composition of the present invention, the form can conform to a livestock meat, chicken meat, and/or minced fish processed food product. That is, examples of the first processed protein-containing composition of the present invention include meat-like processed food products (referring to food products imitating livestock meat, chicken meat, and/or minced fish processed food products). More preferably, examples of the first processed protein-containing composition of the present invention include livestock meat and/or chicken meat-like processed food products (referring to food products imitating livestock meat and/or chicken meat processed food products). Such a livestock meat and/or chicken meat processed food product may be any food product that is cooked by molding and heating a meat type using the livestock meat and/or the chicken meat, and specific examples thereof include hamburg steak, meat balls, patty, meat loaf, minced-meat cutlet, Chinese dumpling and the like.

In the case of using an untextured form as the plant protein-containing composition that is a material for the first processed protein-containing composition of the present invention, examples of the specific form of the first processed protein-containing composition of the present invention include a plant alternative milk, an alternative yogurt, an alternative cheese, an alternative ice cream, and the like, and a plant alternative milk and an alternative yogurt are preferable.

Examples of the specific form of the second processed protein-containing composition of the present invention include a plant alternative milk, an alternative cream, an alternative ice cream, an animal dairy product (animal milk, cream, or ice cream) and the like.

Examples

Hereinafter, the present invention will be specifically described by means of Examples; however, the present invention is not to be construed as being limited to the following Examples.

[A. Protein-Containing Composition]

Protein materials shown in Table 1 were used.

[Table 1]

| | Product name | Plant protein | Protein content | Manufacturer |
|---|---|---|---|---|
| Plant protein material | SOYPRO | Soybean protein | 50 wt% or more | J-OIL MILLS, INC. |
| | Propulse | Pea protein | 80 wt% or more | Roquette Japan K.K. |
| | Premium oat flour | Oat protein | 13.15 wt% | Slow Food Co., Ltd. |
| Animal protein material | MILEI PROTEIN | Whey protein (whey) | 90 wt% | MORINAGA MILK INDUSTRY CO., LTD. |

[B-1. Protein Deamidase]

As the protein deamidase, a protein glutaminase derived from Chryseobacterium proteolyticum (manufactured by Amano Enzyme Inc.) was used. Hereinafter, this protein deamidase is also referred to as "PG".

The activity value of the protein deamidase was measured by the following method.

To 1 mL of a 0.2 M phosphate buffer (pH 6.5) containing 30 mM Z-Gln-Gly, 0.1 mL of a sample solution containing a protein deamidase was added, the mixture was incubated at 37°C for 10 minutes, and then 1 mL of a 0.4 M TCA solution was added to stop the reaction. As a blank, to 1 mL of a 0.2 M phosphate buffer (pH 6.5) containing 30 mM Z-Gln-Gly, 1 mL of a 0.4 M TCA solution was added, 0.1 mL of a sample solution containing a protein deamidase was further added, and the mixture was left to stand at 37°C for 10 minutes.

The amount of ammonia generated in the reaction solution was measured for the solution obtained above using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).

The activity of the protein deamidase was calculated from the following formula with the amount of enzyme that produces 1 $\mu$mol of ammonia per minute being defined as 1 unit (1 U). In the formula, the reaction solution amount is 2.1, the enzyme solution amount is 0.1, and Df is a dilution rate of the enzyme solution. 17.03 is a molecular weight of ammonia.

Protein deamidase activity (U/mL) = Ammonia concentration in reaction solution (mg/L) $\times$ (1/17.03) $\times$ (Reaction solution amount/Enzyme solution amount) $\times$ (1/10) $\times$ Df    [Mathematical Formula 1]

[B-2. Protease Derived from Genus Chryseobacterium]

As the protease derived from the genus Chryseobacterium, a protease derived from Chryseobacterium proteolyticum was used.

The protease activity value was measured by the following method.

After 5 mL of a 0.6% (w/v) casein solution (0.05 mol/L of sodium hydrogen phosphate, pH 8.0) was warmed at 37°C for 10 minutes, 1 mL of a sample solution containing a protease was added, and the mixture was immediately shaken. After this solution was left to stand at 37°C for 10 minutes, 5 mL of a trichloroacetic acid reagent (containing 1.8 (w/v)% trichloroacetic acid, 1.8 (w/v)% sodium acetate, and 0.33 mol/L acetic acid) was added, the mixture was shaken, and incubated at 37°C for 30 minutes again, and the mixture was filtered. The first filtrate (3 mL) was removed, the next filtrate (2 mL) was weighed, 5 mL of a 0.55 mol/L sodium carbonate reagent and 1 mL of Folin's reagent (1 → 3) were added, and the mixture was shaken well and left to stand at 37°C for 30 minutes. An absorbance AT of this solution (enzymatic reaction solution) at a wavelength of 660 nm was measured using water as a control.

Separately, a solution (blank) was prepared by performing the same operation as in the enzymatic reaction solution except that 1 mL of a sample solution containing a protease was weighed, 5 mL of a trichloroacetic acid reagent (containing 1.8 (w/v)% trichloroacetic acid, 1.8 (w/v)% sodium acetate, and 0.33 mol/L acetic acid) was added, the mixture was shaken, 5 mL of a 0.6% (w/v) casein solution (0.05 mol/L sodium hydrogen phosphate, pH 8.0) was then added, the mixture was immediately shaken and incubated at 37°C for 30 minutes, and an absorbance AB of this solution was measured.

Each of 1 mL, 2 mL, 3 mL, and 4 mL of a 1 mg/mL tyrosine standard stock solution (0.2 mol/L of hydrochloric acid) was weighed, and a 0.2 mol/L hydrochloric acid reagent was added thereto to make 100 mL. Each solution (2 mL) was weighed, 5 mL of a 0.55 mol/L sodium carbonate reagent and 1 mL of Folin's reagent (1 → 3) were added, and the mixture was immediately shaken and incubated at 37°C for 30 minutes. For these solutions, absorbances A1, A2, A3, and A4 at a wavelength of 660 nm were measured using, as a control, a solution obtained by weighing 2 mL of a 0.2 mol/L hydrochloric acid reagent and performing the same operation as described above. The absorbances A1, A2, A3, and A4 were plotted on the vertical axis and the amount ($\mu$g) of tyrosine in 2 mL of each solution was plotted on the horizontal axis to prepare a calibration curve, and the amount ($\mu$g) of tyrosine with respect to the absorbance difference of 1 was determined.

The amount of enzyme that causes an increase in colored materials by Folin's reagent corresponding to 1 $\mu$g of tyrosine per minute was defined as 1 unit (1 U).

[Mathematical Formula 2]

$$\text{Protease activity (U/g, U/mL)} = (AT - AB) \times F \times 11/2 \times 1/10 \times 1/M$$

AT: Absorbance of enzymatic reaction solution

AB: Absorbance of blank

F: Tyrosine amount ($\mu$g) when absorbance difference determined from tyrosine calibration curve is 1

11/2: Conversion coefficient to total liquid amount after stop of reaction

1/10: Conversion coefficient per minute of reaction time

M: Amount (g or mL) of sample in 1 mL of sample solution

[B-3. Protease Derived from Other Bacteria]

As the protease, a protease derived from Bacillus licheniformis and a protease derived from Bacillus amyloliquefaciens were used.

The protease was measured by the method described in the section of [B-2. Protease Derived from Genus Chryseobacterium].

[C. Production of Processed Protein-Containing Composition]

[C-1] First Protein-Containing Composition

The plant protein material shown in the above [A. Protein-Containing Composition] was mixed with water to prepare a liquid plant protein-containing composition (pH: about 7) containing 10 wt% of the plant protein material. A predetermined amount of enzyme shown in Table 2 was added to the plant protein-containing composition, and the mixture was incubated at 50°C for 24 hours. Treatment was performed in a boiling water bath for 10 minutes to perform the step of deactivating an enzyme. After the step of deactivating an enzyme, cooling was performed to room temperature to obtain a processed plant protein-containing composition.

[C-2] Second Protein-Containing Composition

The plant protein material shown in the above [A. Protein-Containing Composition] was suspended in deionized water (final concentration: 10 wt%) and boiled until complete gelatinization, 10 U/g-starch $\alpha$-amylase was then added, and the mixture was incubated at 60°C for 30 minutes to prepare a liquid plant protein-containing composition (pH: about 7). A predetermined amount of enzyme shown in Table 3 was added to the plant protein-containing composition, and the mixture was incubated at 50°C for 2 hours. Thereafter, treatment was performed at 100°C for 5 minutes to perform the step of deactivating an enzyme. After the step of deactivating an enzyme, cooling was performed to room temperature to obtain a processed plant protein-containing composition (processed oat milk).

The animal protein material shown in the above [A. Protein-Containing Composition] was suspended in deionized water (final concentration: 5 wt%) to prepare a liquid animal protein-containing composition (pH: about 7). A predetermined amount of enzyme shown in Table 4 was added to the animal protein-containing composition, and the mixture was incubated at 50°C for 2 hours. Thereafter, treatment was performed at 100°C for 5 minutes to perform the step of deactivating an enzyme. After the step of deactivating an enzyme, cooling was performed to room temperature to obtain a processed animal protein-containing composition.

[D. Amino Acid Analysis]

The processed plant protein-containing composition obtained in the above [C-1] was centrifuged (15,000×g, 10 min) to obtain a centrifugal supernatant. The obtained centrifugal supernatant was filtered using a 0.45 $\mu$m filter to obtain a sample for amino acid analysis. For the sample for amino acid analysis, the amount of free amino acids was analyzed using an amino acid analyzer (amino acid analysis using Agilent 1260 Infinity II LC system) according to the protocol of the analyzer. Specifically, first, free essential amino acids (essential AA: histidine, threonine, valine, methionine, tryptophan, phenylalanine, isoleucine, leucine, lysine) and free non-essential amino acids (non-essential AA: tyrosine, cysteine, aspartic acid, asparagine, serine, glutamic acid, glutamine, proline, glycine, alanine, arginine) were measured. The ratio (%) of essential amino acids to the total amount of free amino acids in the sample for amino acid analysis was calculated.

[E. Foamability]

50 mL of the processed protein-containing liquid composition obtained in the above [C-2] was homogenized at 18,000 rpm for 30 minutes, and immediately transferred to a 100 mL graduated cylinder. The total volume VF0 of the composition including foam was measured, and the "foamability" was calculated using the following calculation formula.

$$[\text{Mathematical Formula 3}]$$

$$\text{Foamability (\%)} = 100 \times (\text{VF0 - 50})/50$$

[F. Foam Stability]

In the same manner as in the test of the above [E. Foamability], 50 mL of the processed protein-containing liquid composition obtained in the above [C-2] was homogenized at 18,000 rpm for 30 minutes, and immediately transferred to a 100 mL graduated cylinder, and the volume VF0 of the total volume of the composition including foam was measured. The volume VF30 of the total volume of the composition after being left to stand for 30 minutes was measured, and the "foam stability" was calculated using the following calculation formula.

[Mathematical Formula 4]

$$\text{Foam stability (\%)} = 100 \times (VF30/VF0)$$

[G. Emulsifiability]

30 mL of the processed protein-containing liquid composition obtained in the above [C-2] and 10 mL of canola oil were mixed and homogenized at 10,000 rpm for 2 minutes to prepare an emulsion composition. 50 μL of the emulsion composition immediately after preparation was added to 5 mL of a 0.1 wt% SDS solution. The turbidity (A0) of was measured at an absorbance of 500 nm, and the "emulsifiability" was calculated using the following calculation formula.

[Mathematical Formula 5]

$$\text{Emulsifiability (m}^2\text{/g)} = (2 \times 2.303 \times A0)/(0.25 \times \text{Protein weight})$$

[Test Example 1]

A processed plant protein-containing composition was prepared by the method of "[C-1] First Protein-Containing Composition" of the above [C. Production of Processed Protein-Containing Composition] using the plant protein material (soybean protein material or pea protein material) shown in Table 1 of the above [A. Protein-Containing Composition]. Note that the added amount of PG per 1 g of soybean protein was 30 U, the added amount of the protease was 39 U, the added amount of PG per 1 g of pea protein was 18.8 U, and the added amount of the protease was 24.4 U.

The processed plant protein-containing composition was subjected to the test of the above "D. Amino Acid Analysis", and the ratio (%) of essential amino acids to the total amount of free amino acids was calculated. The results are shown in Table 2.

[Table 2]

| | | Comparative Example 1 | Comparative Example 2 | Example 1 | Comparative Example 3 | Comparative Example 4 | Example 2 |
|---|---|---|---|---|---|---|---|
| Plant protein material (wt%) | Soybean protein material | 10 w/w% | 10 w/w% | **10 w/w%** | - | - | - |
| | Pea protein material | - | - | - | 10 w/w% | 10 w/w% | **10 w/w%** |
| PG (amount per 1 g of plant protein material) | | 15 U*a | 15 U*a | **15 U*a** | 15 U*c | 15 U*c | **15 U*c** |
| Protease (amount per 1 g of plant protein material) | Derived from *Bacillus licheniformis* | 19.5 U*b | - | - | 19.5 U*d | - | - |
| | Derived from *Bacillus amyloliquefaciens* | - | 19.5 U*b | - | - | 19.5 U*d | - |
| | Derived from *Chryseobacterium proteolyticum* | - | - | **19.5 U*b** | - | - | **19.5 U*d** |
| Water | | Remainder | Remainder | Remainder | Remainder | Remainder | Remainder |
| Total | | 100 w/w% | | | | | |
| Essential amino acid ratio (%) | | 48.2% | 48.1% | **55.9%** | 20.5% | 24.8% | **38.8%** |

*a: 30 U per 1 g of soybean protein, *b: 39 U per 1 g of soybean protein
*c: 18.8 U per 1 g of pea protein, *d: 24.4 U per 1 g of pea protein

As shown in Table 2, it was confirmed that the essential amino acid release amount of the processed plant protein-containing composition was increased by performing the treatment of causing PG and a protease derived from the genus Chryseobacterium to act on a plant protein material, as compared with the case of treating with another protease derived from bacteria.

[Test Example 2]

A processed protein-containing composition was prepared by the method of "[C-2] Second Protein-Containing Composition" of the above [C. Production of Processed Protein-Containing Composition] using the plant protein material (oat protein material) or the animal protein material (whey protein material) shown in Table 1 of the above [A. Protein-Containing Composition].
For the processed protein-containing composition, the tests of the above [E. Foamability], the above [F. Foam stability], and the above [G. Emulsifiability] were performed. The results are shown in Tables 3 and 4.

[Table 3]

| | | Comparative Example 5 | Example 3 | Example 4 | Example 5 |
|---|---|---|---|---|---|
| Protein material (wt%) | Oat protein material | 10 w/w% | 10 w/w% | 10 w/w% | 10 w/w% |
| PG (amount per 1 g of protein) | | 10 U | 10 U | 10 U | 10 U |
| Protease derived from *Chryseobacterium proteolyticum* (amount per 1 g of protein) | | - | 20 U | 50 U | 100 U |
| Water | | Remainder | Remainder | Remainder | Remainder |
| Total | | 100 w/w% | | | |
| Emulsifiability (m$^2$/g) | | 59.7 | 61.7 | 64.0 | 68.1 |

[Table 4]

| | | Comparative Example 6 | Example 6 | Example 7 | Example 8 | Example 9 | Example 10 |
|---|---|---|---|---|---|---|---|
| Protein material (wt%) | Whey protein material | 5 w/w% | 5 w/w% | 5 w/w% | 5 w/w% | 5 w/w% | 5 w/w% |
| PG (amount per 1 g of protein) | | 10 U | 10 U | 10 U | 10 U | 10 U | 10 U |
| Protease derived from *Chryseobacterium proteolyticum* (amount per 1 g of protein) | | - | 1 U | 10 U | 20 U | 50 U | 100 U |
| Water | | Remainder | Remainder | Remainder | Remainder | Remainder | Remainder |
| Total | | 100 w/w% | | | | | |
| Foamability (%) | | 143.3 | 150.0 | 193.3 | 213.3 | 223.3 | 233.3 |
| Foam stability (%) | | 74.9 | 80.0 | 92.7 | 96.8 | 95.9 | 92.8 |
| Emulsifiability (m$^2$/g) | | 30.5 | 37.1 | 39.0 | 42.8 | 51.2 | 59.9 |

As shown in Tables 3 and 4, it was confirmed that the foamability, foam stability, and emulsifiability of the processed protein-containing composition were improved by performing the treatment of causing PG and a protease derived from the genus Chryseobacterium to act on a protein material. Note that, in the processed protein-containing composition of Table 3, the essential amino acid release amount also increased as in Table 2.

**Claims**

1. An agent for improving an essential amino acid release amount of a plant protein-containing composition, the agent comprising a protein deamidase and a protease derived from the genus Chryseobacterium.

2. The agent for improving an essential amino acid release amount of a plant protein-containing composition according to claim 1, wherein the protein deamidase is a protein glutaminase.

3. The agent for improving an essential amino acid release amount of a plant protein-containing composition according to claim 1, wherein the plant protein is a protein of a plant selected from the group consisting of pulses, cereals, and nuts and seeds.

4. A modifier for a protein-containing composition, the modifier comprising a protein deamidase and a protease derived from the genus Chryseobacterium, wherein the modification is an improvement in foamability, foam stability, and/or emulsifiability.

5. A method for improving an essential amino acid release amount of a plant protein-containing composition, the method comprising a step of causing a protein deamidase and a protease derived from the genus Chryseobacterium to act on a plant protein-containing composition.

6. A modification method for a protein-containing composition, the modification method comprising a step of causing a protein deamidase and a protease derived from the genus Chryseobacterium to act on a protein-containing composition, wherein the modification is an improvement in foamability, foam stability, and/or emulsifiability.

7. A method for producing a processed plant protein-containing composition with an improved essential amino acid release amount, the method comprising a step of causing a protein deamidase and a protease derived from the genus Chryseobacterium to act on a plant protein-containing composition.

8. A method for producing a processed protein-containing composition with improved foamability, foam stability, and/or emulsifiability, the method comprising a step of causing a protein deamidase and a protease derived from the genus Chryseobacterium to act on a protein-containing composition.

9. A processed plant protein-containing composition with an improved essential amino acid release amount, the processed plant protein-containing composition being obtained by a method including a step of causing a protein deamidase and a protease derived from the genus Chryseobacterium to act on a plant protein-containing composition.

10. A processed protein-containing composition with improved foamability, foam stability, and/or emulsifiability, the processed protein-containing composition being obtained by a method including a step of causing a protein deamidase and a protease derived from the genus Chryseobacterium to act on a protein-containing composition.

## INTERNATIONAL SEARCH REPORT

| International application No. |
|---|
| **PCT/JP2024/021775** |

**A. CLASSIFICATION OF SUBJECT MATTER**

*C12P 1/04*(2006.01)i; *A23J 3/34*(2006.01)i; *A23L 5/00*(2016.01)i; *A23L 33/175*(2016.01)i; *C12N 9/52*(2006.01)i; *C12N 9/80*(2006.01)i; *C12P 13/04*(2006.01)i

FI: C12P1/04 Z; A23L33/175; A23L5/00 M; A23J3/34; C12P13/04; C12N9/52; C12N9/80

According to International Patent Classification (IPC) or to both national classification and IPC

**B. FIELDS SEARCHED**

Minimum documentation searched (classification system followed by classification symbols)

C12P1/04; A23J3/34; A23L5/00; A23L33/175; C12N9/52; C12N9/80; C12P13/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2000-50887 A (AMANO PHARMACEUT CO., LTD.) 22 February 2000 (2000-02-22) claims, paragraphs [0001]-[0008], [0036]-[0059], example 20 | 1-8 |
| X | | 9, 10 |
| A | WO 2022/102723 A1 (AMANO ENZYME INC.) 19 May 2022 (2022-05-19) in particular, claims, paragraphs [0001]-[0004], [0036]-[0059], examples | 1-10 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 August 2024** | **13 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/021775**

| Patent document cited in search report | | | Publication date (day/month/year) | Patent family member(s) | | | Publication date (day/month/year) |
|---|---|---|---|---|---|---|---|
| JP | 2000-50887 | A | 22 February 2000 | US claims, paragraphs [0001]-[0009], [0087], [0092], example 20 | 2004/0072318 | A1 | |
| | | | | EP | 976829 | A2 | |
| WO | 2022/102723 | A1 | 19 May 2022 | US claims, paragraphs [0001]-[0004], [0036]-[0059], examples | 2024/0016187 | A1 | |
| | | | | EP | 4245149 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000050887 A
- JP 2001218590 A

- WO 2006075772 A

**Non-patent literature cited in the description**

- *Journal of Japanese Society of Food and Nutrition*, vol. 20 (4), 259-266